# EUROPEAN PATENT APPLICATION

(11) **EP 0 921 488 A1**
(43) Date of publication of application: **09.06.1999**
(21) Application number: 98310032.2
(22) Date of filing: 08.12.1998
(51) Int. Cl.: G06F 19/00

(54) **Automated database-oriented prescription drug ordering system**

(30) Priority: 08.12.1997 US 986805
(71) Applicant: Cohen, Kopel H., Longboat Key, FL 34228 (US)
(72) Inventor: Cohen, Kopel H., Longboat Key, FL 34228 (US)
(74) Representative: Cross, Rupert Edward Blount

(57) **Abstract**

A system for automating activity relating to ordering health-related products using: a central database subsystem which stores patient-related information and information related to ordering health care products, a provider subsystem which is accessible to health care provider, and a product order subsystem which is accessible to an entity which processes or fills orders for health related products. Based on the information stored in the database of the central database subsystem, the system automatically transmits information to the provider subsystem. The health care provider can then analyze the information transmitted for the need to take action relating to ordering health care products and communicate information relating to that need back to the central database subsystem. The central database subsystem can then automatically transmit information relating to ordering health related products to the provider subsystem.

## Description

### Field of Invention

The present invention is directed generally to a method and apparatus for ordering health-related products, including prescription drugs, in an automated fashion. More particularly this invention is directed to a method and system for automatically taking action with respect to ordering health-related products, based on the health status and prescription activity of outpatients.

### Copyright Notice

A portion of the disclosure of this patent document contains material which is subject to copyright protection. The copyright owner has no objection to the facsimile reproduction by anyone of the patent document or patent disclosure as it appears in the Patent and Trademark Office, patent file or records, but otherwise reserves all copyright rights whatsoever.

### Background of the Invention

Numerous health care resources are dedicated to providing timely medical advice which is based on the most up-to-date information available. This is especially so with respect to placing new orders for and refilling previous orders for health-related products, such as prescription drugs and home care measuring devices (e.g. glucose measuring strips). Before initial orders are placed, patients typically consult with health care providers who prescribe the health-related product. The patient often obtains the prescribed product from another health care provider, such as a pharmacist, for example. The prescribing health care providers must then periodically monitor the health status of the patient to determine the effectiveness of the prescribed product, the need to either modify or refill the current prescription, or issue a new prescription.

Much of the cost of providing health care is associated with time spent by medical personnel consulting with patients. Whereas equipment is relatively easy to procure, it is costly to provide the extensive training and experience required by health care professionals to enable them to provide quality health care. The growing demand for medical services at a reasonable cost has placed unprecedented demands on the health-care provider.

The process also burdens patients, especially those treated on an outpatient basis. Outpatients usually reside at locations away from their health care provider. Thus, the health care provider treating or consulting with an outpatient has far less opportunity to monitor the health status of the outpatient patient on a day-to-day basis. Moreover, an outpatient may be required to see a doctor on a regular basis, often for simple and routine tests. In such a case, the outpatient must travel to the doctor's office or to a hospital, wait to be seen by the relevant health care provider, have the tests performed, pick-up any prescribed health-related products, and travel home. This is an inconvenient way of monitoring the health of an outpatient which often does not assist the outpatient in the recovery process. Further, it is an unreliable way of monitoring outpatient health, because it depends largely on the outpatient to be proactive in seeking consultation from a health care provider, ordering any prescribed health-related products, and complying with instructions for taking or using the prescribed products.

Medical studies have shown that a large percentage of patients do not comply with instructions for taking or using prescribed health-related products. With respect to prescription drugs, for example, many patients either forget to take drugs as prescribed or to refill the prescription when necessary. Patients also fail to schedule appointments in order to obtain new prescriptions, as refill authorizations, which are not indefinite, expire. This patient noncompliance, which often exacerbates illness and sometimes causes death, typically goes unchecked by health care providers who await indications from patients that they are experiencing health problems.

In short, the ability of health care providers to provide effective, affordable health care is severely hampered by the amount of resources those providers must expend in ordering health-related products, and by the degree to which they must rely on significant effort of patients in complying with medical instructions and relaying their health status. A system is needed that will help ease the burdens on both health care providers and the patients in ordering health-related products.

Prescription compliance systems, directed at simply reminding patients and health care providers when a prescription needs to be refilled, are known in the art. For example, United States Patent No. 4,766,542, describes a system for automatically contacting the customers of a particular pharmacy to remind them that their prescription needs to be filled. Systems for dispensing prescription drugs within a health care institution, notifying nurses when medications should be given, are also known in the art. (See, e.g. United States Patent No. 4,847,764).

### Summary of the Invention

The present invention is directed to a method and system for automatically triggering the process for ordering health-related products for patients, in particular outpatients, based on updated information concerning the patient's health status. The invention preferably uses telecommunications hardware systems already likely to exist in a health care provider's office, such as, for example, a telephone or personal computer with a modem, and a memory device such as a database of a software program run on a personal computer.

Distinct from systems which simply remind patients of the need to refill prescriptions, and also from those which notify doctor of their patients' refill activity, the present invention facilitates the entire process of ordering health related products. (United States Patents Nos. 4,766,542 and 5,612,869 describe systems for reminding patients of their need to refill prescriptions and incorporated herein for their teachings).

As used herein, the term "provider" or "health care provider" includes doctors, pharmacists, psychologists, HMOs, hospitals, health clinics, managed care entities, and the like.

The representative embodiment of the present invention can be regarded as having three subsystems, namely, a central database subsystem, a health care provider subsystem, and a product order subsystem.

The central database subsystem is capable of receiving, storing and processing patient-related health information provided by one or more sources, and transmitting information to the appropriate health care providers. In the representative embodiment, the central database subsystem comprises at least one computer processor coupled to a communications line and a memory storage device.

The memory storage device of the central database subsystem is preferably a database which includes associated controlling software such as a DBMS. The database contains patient-related health information as well as information related to placing an order for prescribed health-related products. The stored patient-related health information relates to the need of patients for prescribed health-related products and includes: the names and contact information for patients, the names and contact information for doctors and other health care providers, disease and treatment histories for patients, medications and health-products taken or used by patients, dosages of medications taken or quantities of health-products used, the time when new medications or products will be needed, information relating to patients' health-status, and information relating to patients' product ordering preferences. The stored information relating to ordering health-related products includes: names and contact information for entities that treat patients and entities that process product orders, information relating to the products that those entities carry.

The stored information relating to products can include product compatibility information. This information can be used by the system to identify potential contraindications when products are prescribed. This particularly useful in the case of prescription drug interactions.

The database is preferably updatable by any of the means known in the art including data entry, batch processing, or by receiving information from remote sites over a communications line. With the ability to receive updated information, the database of the central database subsystem can reflect the most current health status of patients. Health care providers can then base decisions concerning ordering health-related products on the up-to-date health status of patients, without taking the time or incurring the expense of personally consulting with patients.

In the representative embodiment, the health care provider subsystem is a device capable of receiving information and instructions from the central database subsystem and also capable of sending information to the central database subsystem over the Internet, via e-mail for example. Typically, the health care provider subsystem comprises a computer and modem, but may also comprise a touch-tone telephone, television with user interface, or facsimile device.

In the representative embodiment, the product order subsystem is a device capable of receiving information and instructions from the central database subsystem over the Internet. The product order subsystem is used to provide access to or communicate with entities which fill or process orders for health related products. Such entities include direct mail houses, physicians and drug stores. Patients may also be such entities in that they process medication instructions from their health care provider. Instructions to reduce dosage or discontinue taking a prescription can be communicated directly to a patient through a product order subsystem to which the patient has access.

Typically, the product order subsystem comprises a computer and modem, but may also comprise a telephone, television, or facsimile device. In some instances, the health care provider subsystem will also perform the function of the product order subsystem.

Typically, the central database subsystem is located in a central location so as to be accessible by communication device to all health care providers with patients whose information is stored in the database. Since the central monitoring subsystem could be used and shared by a number of health care providers, it could be located in a remote location, accessible by modem, WAN or the Internet. Typically, the database contains the information of numerous patients who use numerous health care providers. As a result, there are typically multiple health care provider subsystems to provide access to the many providers. Similarly, there are typically numerous product order subsystems to access or communicate with the numerous entities that must be contacted in ordering health-related products.

The representative embodiment of the present invention operates as follows: The central database subsystem determines, based on the information stored in the database, the patients whose health care providers should be contacted regarding ordering health-related products. The central database subsystem also determines when that contact should be made and, at the appropriate time, automatically performs the communications necessary to contact the appropriate health care providers and order products if necessary. The central database subsystem contacts a particular health care provider by using a transmission device to communicate with a health care provider subsystems to which that provider has access. The health care provider can then access the patient-related information communicated to the provider subsystem and determine whether any new health-related products need to be ordered, or whether any current prescriptions need to be refilled, discontinued, or otherwise modified or acted upon. The provider then indicates whether or not an order needs to be placed or modification needs to be made, indicates the details of and authorizes the placement of any such order or modification, and transmits the indications and authorizations to the central database subsystem. The central database subsystem then automatically communicates with the appropriate product ordering subsystems to place any orders and communicate any modifications indicated by the provider.

The present invention is particularly useful for automating the ordering of prescription drug refills. For example, the central database subsystem can generate a list of patients whose time for refilling a medication prescription is approaching and, for each patient, contact the health care provider subsystem of the provider identified in the database as having authority to refill the prescription. As further example, the central database subsystem can generate a list of patients whose health care status indicates that the previously prescribed drug is no longer effective, and communicate the lack of effectiveness to the appropriate health care provider subsystems. The providers can then analyze the information received, determine any action that needs to be taken with respect to prescribing health related products, and transmit authorization to place new or cancel old orders to the central database subsystem. The central database subsystem can then communicate with the appropriate product order subsystem to place the appropriate order.

### Brief Description of the Drawings

Figure 1 is a block diagram of the three main subsystems of the present invention.
Figure 2 is a block diagram illustrating in further detail the components of the three subsystems of Fig. 1.
Figure 3 is a block diagram illustrating the three main subsystems of the present invention, with the central database subsystem coupled to a data input device.
Figure 4 is a block diagram illustrating in further detail the components of the three subsystems of an alternate embodiment of the present invention, with the central database subsystem including a voice generator and a DTMF modem.
Figure 5 illustrates an example of a relational database table containing patient history, as recorded in the database of Fig. 2.
Figure 6 illustrates examples of relational database tables containing prescription history, health care provider information, and product order entity information, as recorded in the database of Fig. 2.
Figure 7 illustrates examples of a relational database table containing patient information, as recorded in the database of Fig. 2.

### Detailed Description

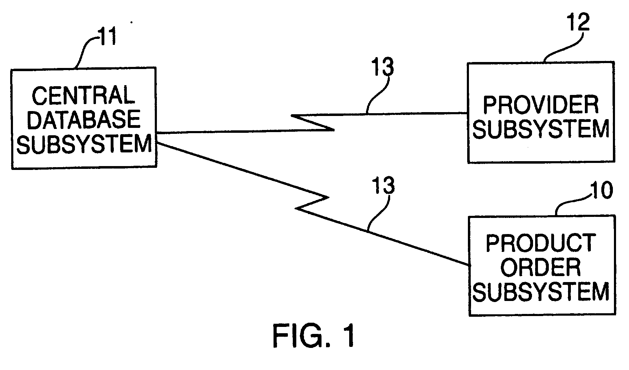

Referring now to the drawings wherein like reference numerals designate like parts in the several figures, and initially to Fig. 1, there is illustrated a representative embodiment of an automatic prescription drug ordering system comprising three subsystems, namely, a central database subsystem 11, a provider subsystem 12, and a product order subsystem 10. The central database subsystem 11 stores information relating to the health status of patients, processes that information and communicates information relating to the patients' need for health care products to health care providers by transmitting that information to the providers' provider subsystems 12. Based on the information communicated to their provider subsystem 12, providers then determine whether any action should be taken to place a new order, or modify or cancel a previous order of health-related products for the patients whose information they received. If any such actions should be taken for any of the patients, the provider provides the necessary order information and transmits that information back to central database subsystem 11. The central database subsystem 11 then processes the information received from the provider subsystem 12 and, if necessary, places new orders or communicates the need to modify previous orders for health-related products by contacting the appropriate product order subsystems 10.

Fig. 1 shows only one provider subsystem 12 and one product order subsystem 10. However, the present invention is designed to enable contacting many providers and many entities that process product orders. Thus, there will be many provider subsystems 12, for example, one for each provider with a patient listed in the database, each provider subsystem 12 coupled to the central database subsystem 11 via the communications system 13. There will also be many product order subsystems 10, each coupled to the central database subsystem 11 via the communications system 13.

The central database subsystem 11 is located so as to be readily accessible to one or more health care providers through one or more provider subsystems 12. The central database subsystem 11 is also located so as to be readily accessible to one or more entities which process orders for health-related products, through one or more product order subsystems 10.

The central database subsystem 11 is coupled to each provider subsystem 12 and each product order subsystem 10 by a communications system 13, such as, for example, a public telephone link, satellite link, radio-frequency link, infra-red link, facsimile link, fiber-optic link, coaxial cable link, or television link.

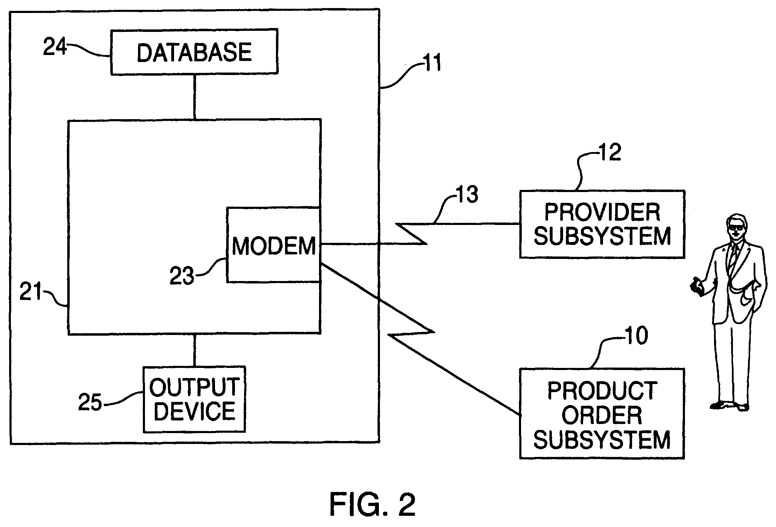

Fig. 2 illustrates the representative embodiment of the central database subsystem 11 of the present invention. The central database subsystem 11 comprises a computer which can be a minicomputer, microcomputer, or mainframe computer. The computer which is the central database subsystem 11 comprises a computer processor 21, such as, for example, an Apple Macintosh computer, a SUN brand workstation or an IBM personal computer with a 486 Intel processor. The computer processor 21 includes a transmission device which is a modem 23 and a memory storage device, which is preferably a database 24 and which will, for purposes of ease of description, be referred to as such. The computer processor 21 can also be coupled to an output device 25, such as a monitor or a printer, and to a voice generator 22. The modem 23 is coupled to the communications system 13, as is the voice generator 22 if present.

The computer processor 21 is capable of executing software programs, such as DBMS programs and other programs capable of carrying out the operations involved in automating the process of ordering health-related products. The computer processor 21, in conjunction with the software programs, is capable of sending information to and receiving information from both the provider subsystems 12 and the product order subsystems 10 via the modem 23. In addition, the computer processor 21 is capable of actuating the voice generator 22, when that component is present.

The database 24 of central database subsystem can be any memory storage device such as ROM, RAM, disk drive (such as floppy, hard, or optical disk drive), or other memory storage device. The database 24 includes a memory management or control system, such as a DBMS, which can be used to access the stored information.

The database 24 in the representative embodiment is a relational database that is used to store patient-related health information, as well as information relating to ordering health-related products. The stored patient-related health information is particularly that information relating to the need of patients for prescribed health-related products and includes: the names and contact information for patients, the names and contact information for doctors and other health care providers, disease and treatment histories for patients, medications and health-products taken or used by patients, dosages of medications taken or quantities of health-products used, the time when new medications or products will be needed, and information relating to patients' health-status. The stored information relating to ordering health-related products includes: names and contact information for entities that process product orders and information relating to the products that those entities carry.

In addition, the database can have information relating to health related products, such as their compatibility with other health related products. For example, the database can have a table with a row for each health related product which indicates, for each product, other products with which that product should not be used. This information can be used by the system to identify potential contraindications in prescribing products and communicate that information to the health care providers.

The database can also contain product price information which can be used by the health care provider to determine which product order entity offers the best purchase price. The prices offered each product order entity can be stored in tables database 24. Upon receiving authorization to order from a health care provider, the system can query the database table for the entity offering the best price for that product. The system can then transmit the order information to that entity's product order subsystem 10.

In addition, the database 24 can contain information relating to insurance companies. The central database subsystem can use this information to transmit product order information to insurance companies as well. The insurance company information can be stored in tables of the database 24.

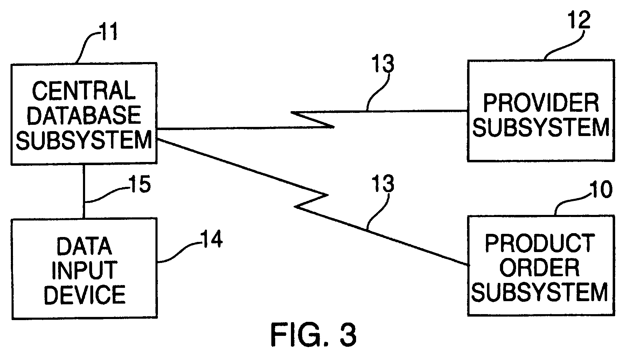

The database 24 can be populated with the stored information in a number of different ways. Figure 3 illustrates the automatic ordering system of the present invention with the central database subsystem 11 coupled to a data input device 14. The data input device 14 can be a keyboard, a separate computer system, a touch-tone-telephone, data tape feed, or other device which allows data to be sent to the central database subsystem 11. A coupler 15 is used to communicate the input data to the central database subsystem 11 for storage in the database 24. The coupler can be any device capable of allowing or enabling communication between the data input device 14 and the central database subsystem 11.

Methods for entering new data into a database using a data input device 14 are well known in the art and can be used to populate the database 24. They can also be used to periodically update the database 24. United States Patent No. 5,633,910 describes a method and apparatus for monitoring the health status of patients and, in particular, a method and system utilizing a telecommunications system and a Dual Tone Multifrequency ("DTMF") decoder to monitor the health status of outpatients. The disclosed method entails updating patient information stored in a database using touch-tone-telephony. The apparatus and methods described therein can be used to populate and update information stored in the database 24 of the present invention. The contents of that patent are incorporated herein for their teachings.

Different subsets of information can be input or updated at different times. For example, patient name and contact information can be initially entered via keyboard or data tape feed and updated only when a patient indicates that some of that information has changed. As another example, health status information can be updated at regular intervals and, in addition, each time a patient transmits health status information, using the method and apparatus described in United States Patent No. 5,633,910 or otherwise. As further example, information relating to prescription drugs can be input or updated each time a prescription is filled, or on a daily basis or some other periodic basis. As yet another example, product price information can be periodically updated, for example upon receipt of price information from a product order subsystem or upon receipt of a data feed from a price reporting service.

As is well-known in the art, stored data can be retrieved in a different manner than that in which it is stored. Data is typically stored in manner that allows efficient retrieval during processes that are run most often and those that take the most time to perform. However, data can be retrieved from memory storage devices and transmitted in a different order than the order in which it is stored. In addition, mere subsets of data can also be retrieved and transmitted. Stored data can also be manipulated prior to transmission. Thus, the data or information communicated from the central database subsystem 11 to the provider subsystem 12 and the product order subsystem 10 can be in a form different from that in which the data is stored in the database 24. For example, patient-specific prescription activity information can be stored in a relational database in alphabetical order by patient name. It can, however, be retrieved such that information can be transmitted in order of urgency, or in descending order of need for a prescription.

The central database subsystem 11, using the computer processor 21 and the database 24 (including the software which controls and accesses them) processes the information stored in the database, analyzing that data relevant to a determination of whether patients need health-related products. If the analysis indicates that a particular patient's health care provider should be contacted concerning the patient's need for a health-related product, the central database subsystem 11 can automatically contact the provider subsystem 12 of the appropriate health care provider using the communications system 13. Upon receiving an indication from the provider that an order should be placed, the central database subsystem 11 can also automatically contact the appropriate product order subsystem 10.

The provider subsystem 12 of the representative embodiment is a computer with a graphical user interface (GUI), such as a personal computer. The provider subsystem 12 is capable of receiving a transmission from the central database subsystem 11 over the communications system 13. The provider subsystem 12 has software capable of displaying the transmitted information to the health care provider. Using the GUI, the provider can then indicate whether any of the patients whose information was received are in need of any health-related products, indicate the type and quantity of any needed products, and authorize the ordering of any products if necessary. The provider can then transmit these indications and authorizations back to the central database subsystem 11 via the communications system 13.

The use of computers with GUI's is well known in the art. The use of software programs in connection with such computers to control the receipt of data over a communications system, and the display of the data for an end user such as a health care provider is also well known in the art.

The central database subsystem 11 receives the indications and authorizations transmitted from the provider subsystem 12 and can store a record of the indication and authorization in the database 24. The central database subsystem 11 can also automatically place the indicated orders for health-related products by transmitting order information to the appropriate product order subsystems 10.

The product order subsystem 10 is a device capable of receiving orders transmitted from the central database subsystem 11. The product order subsystem 10 may be, for example, a facsimile, telephone or personal computer located at a site accessible to an entity which files or processes orders, such as a physician, drug store, or direct mail box. The product order subsystem can also be located at a site accessible to a patient who can then receive communications containing instructions from health care providers.

The product order subsystem 10 need only be able to receive the data sent via the communications system 13. In an alternate embodiment, the product order subsystem 10 is capable of transmitting error or problem codes back to the central database subsystem 11. In this alternate embodiment, the product order subsystem 10 is, for example, a touch-tone telephone or personal computer.

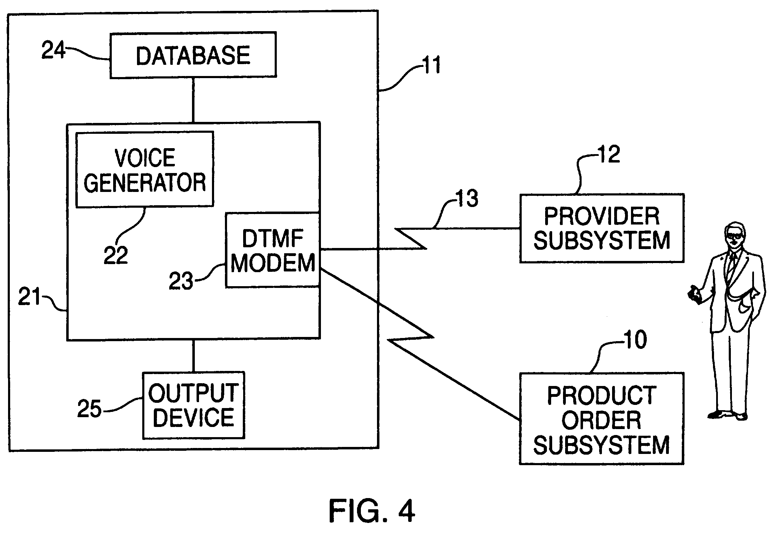

In an alternate embodiment of the automatic prescription drug ordering system of the present invention, the communication from the central database subsystem 11 to the provider subsystems 12 and product order subsystems 10 is performed using a telecommunications system and DTMF decoder. In this embodiment, the computer processor 21 of the central database subsystem 11 includes a voice generator 22, and the modem 23 is a DTMF modem, as shown in Figure 4. As is known in the art, the DTMF modem is capable of receiving the dual tones generated by a touch tone telephone keypad that have been transmitted over the telephone line and translating the tones into characters recognizable by the computer processor. In the alternate embodiment, the DTMF modem 23 is a ACC303800 Sporster FAX/modem or Digicom Systems Connection's +14.4 FAX/modem, both with DTMF dialers/decoders fax and modem chips.

In this alternate embodiment, the provider subsystem 12 is a device capable of receiving and generating dual tones. Touch-tone telephones and computers with modems are examples of such devices. Similarly, the product order subsystem 10 is a device capable of receiving dual tones. The equipment used in this alternate embodiment is readily available commercially, is inexpensive, and is easy to use.

United States Patent No. 5,633,910, described supra, discloses in detail a method and apparatus for communicating between a DTMF modem and a touch-tone telephone over telecommunications lines. The contents of that reference are incorporated herein for those teachings.

The method of automatically ordering health-related products according to the present invention operates as described below. For purposes of explanation only, the method will be described using the example of automatically ordering prescription drugs. The method of the present invention can be used to automatically order any health-related product.

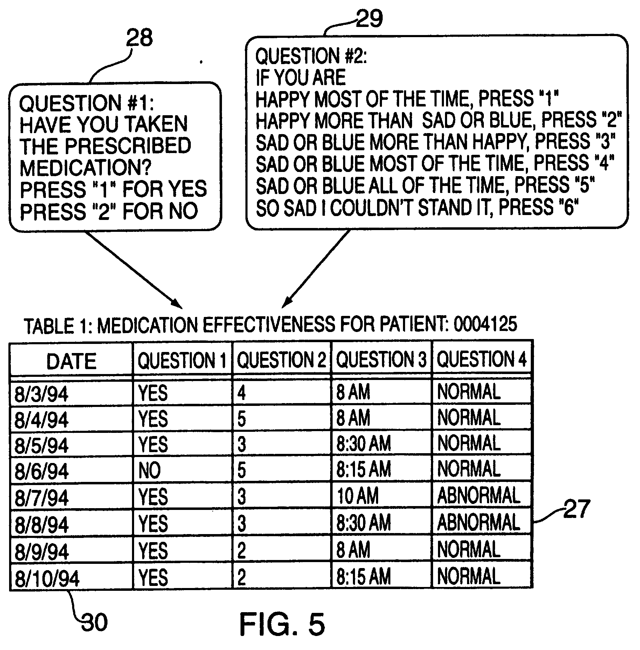

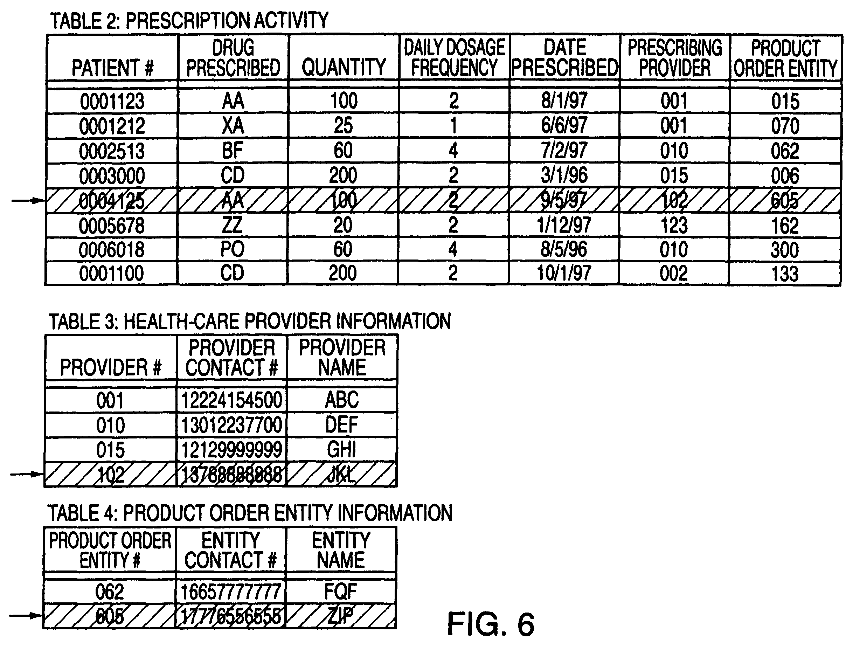

Figures 5 and 6 illustrate representations of data tables which can be stored in the relational database 24 of the representative embodiment of the present invention. More generally, they represent the type of information which can be stored in the database 24.

Table 1 on Figure 5 illustrates a table which stores health status information of a particular patient, patient number 0004125. Table 1 contains patient-specific data. More specifically, the table stores information relating to the effectiveness of prescribed medication which the patient has been taking. The data in the table reflects whether the patient has been taking the medication, and if so, what effect the information has had on the patient. In the case of patient 0004125, the table reflects how effective the prescribed medication has been in controlling the patient's mood.

Table 2 on Figure 6 illustrates a table which stores information relating to the prescription activity of several patients. The table contains the following information: patient identification number, prescription drug identification code, quantity of drug prescribed, daily dosage frequency, date the drug was prescribed, the identification number for the prescribing health care provider, and the identification number for the entity which filled the order for the prescribed drug. Note that for patient number 0001212, the identification numbers for the prescribing health care provider is identical to that of the entity which filled the prescription order. That record of Table 2, that is the record for patient number 0001212, illustrates that the prescribing provider can also be the entity which fills the order. This especially common with full-service HMO centers such as those operated by HIP™.

Table 3 on Figure 6 illustrates a table which stores information relating to health care providers. The table contains the following information: health care provider identification number, health care provider contact number, and health care provider name.

Table 4 on Figure 6 illustrates a table which stores information relating to entities which fill orders for health care products. The table contains the following information: entity identification number, entity contact number, and entity name.

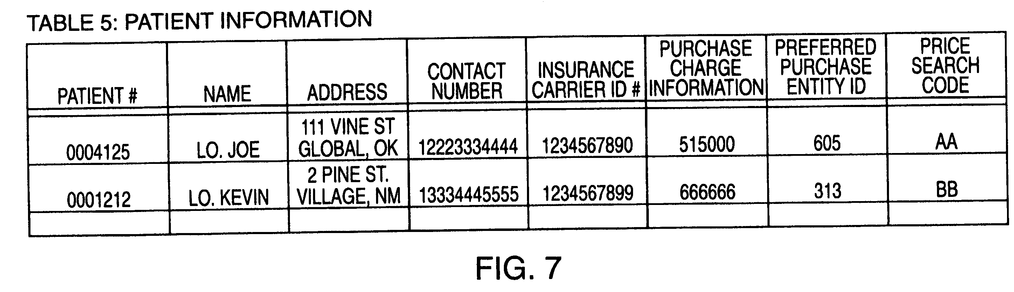

Table 5 on Figure 7 illustrates a table which stores information relating to patients. The table contains basic patient identification information such as patient identification number, name, address and contact number. The table also contains information relating to patient insurance carrier such as insurance carrier name, insurance carrier identification number and insurance carrier contact number. This information can be used by the system to further facilitate product ordering by transmitting purchase information to insurance carriers upon or after receiving authorization from health care providers to place orders. The table can also contain purchase-related information such as: patient charge information, which includes a charge number which can be used to purchase any ordered health related product; the preferred purchase entity, which indicates the preferred purchase order entity that the patient would like to fill any product orders; and the price search code, which indicates whether patient would like the system to place product orders with the product order entity offering the best price for the product to be ordered. If this code indicates that the patient would like such a search, the system can query the database for price information, contact any price order entities for price updates if necessary, and then contact the entity offering the best price.

The central database subsystem 11 queries the database 24 to located patient information which should be brought to the attention of health care providers. This querying is performed using the computer processor 21 and the DBMS. The central database subsystem 11 can, for example, query the database 24, retrieving the information stored in Table 2. From that information, the central database subsystem 11 can process each patient record sequentially determining, for each record, the date on which the patient's prescription needs to be refilled. This date can be calculated using the quantity, daily dosage frequency, and date prescribed fields. For those patients whose date for refilling prescription has either past or is approaching, the central database subsystem can use the prescribing provider identification number in Table 2, to locate the record containing information for that provider in Table 3.

The central database subsystem 11 can then use the provider contact number from Table 3 to contact the provider subsystem 12 for the prescribing provider. The central database subsystem 11 utilizes the modem 23 to communicate with the appropriate provider subsystem through the communication system 13. As part of the information transmitted, the central database subsystem 11 can send information from Table 1 concerning the patient's health status, and information from Table 2 concerning the patient's prescription activity.

The health care provider can then access the information through the GUI of the provider subsystem 12. The provider can not only access information relating to need to place a new order or modify a previous order, but also information relating to whether the patient has actually been taking the medication (or using the product) and, if so, whether the information has been effective.

Based on this information, the provider can determine whether to authorize a refill of the prior prescription, modify the dosage of a prior prescription, discontinue the current prescription, or prescribe another medication. The provider can also indicate the type, quantities, dosages, and prescription instructions for any medication that should be ordered for the patient. In the alternative, the provider can also simply indicate that the transmission from the central database subsystem was received and reviewed, and that no order need be placed. In any event, the provider has updated patient-related information on which to base the decision whether or not to order, all without having to personally consult with the patient, or to even take any action to initiate the ordering process.

When the health care provider determines, based upon the information received from the central database subsystem 11, that the patient is being adversely effected by a prescribed drug, the health care provider can contact the patient and reduce the dose of the medication or change the medication to reduce the deleterious effect. Alternatively, the system of the present invention, upon receiving authorization from the provider to do so, can use the patient contact information stored in the database 24 to contact the patient directly and communicate the information received from the health care provider.

The provider then makes any indications and authorizations using the GUI of the provider subsystem, then transmits any indications and authorizations to the central database subsystem over the communications system 13. The central database subsystem 11 receives the transmission from the provider subsystem 12 and, based on the data containing the provider's indications and authorizations, determines whether an order for prescription drug needs to be placed. If so, the central database subsystem determines the appropriate product order entity and the appropriate product order subsystem 10. The provider can indicate a particular product order entity using the GUI of the provider subsystem 12. Alternatively, the central database subsystem 11 can query the database 24 using the patient identification number and identification code of the drug prescribed, from Table 2 for example, and locate the identification number for the provider that last prescribed that medication to that patient. The central database subsystem can then locate the appropriate provider contact number from Table 2 of the database 24, and transmit an order for the prescribed medication to the appropriate product order subsystem 10. The product order entity can then place the order.

In an alternate embodiment of the method according to the present invention, dual pulse tones are used to communicate between the central database subsystem 11 and the provider subsystem 10 over the communication system 13. The details of the method for using a DTMF modem and touch-tone telephones for such communication is fully described in detail in United States Patent No. 5,633,910, which is incorporated by reference for those teachings.

The present invention is very useful in facilitating the communication between physicians and pharmacists. This communication is important because pharmacists often have more detailed knowledge of medications and possible contraindications than do physicians. By facilitating the interaction between physicians and pharmacists, the system provides the synergy between the physicians' detailed knowledge of the patient medical histories and the pharmacists' detailed knowledge of medication.

The database 24 of the central database subsystem 11 can store the information needed to make physician-pharmacists communication not only better, but automatic. In essence, the system of the present invention allows physicians and pharmacists to share the information stored in database 24, and provide patients with better care, advice and service by automating communication between the two entities.

One method of communication between the physician and pharmacist using the system of the present invention is as follows. The pharmacist who fills the prescription for a patient communicates information relating to that prescription to the central database subsystem 11 using a data input device 14 and a coupler 15. The information can include a patient identifier such as name and patient number, the medication prescribed, the dosage of the prescribed medication and the number of doses dispensed. The information is stored in the database 24 and then used, along with other data stored in the database 24, to determine whether a physician should be contacted regarding the patient. If so, the central database subsystem 11 automatically contacts the provider subsystem 12 for the appropriate physician, transmitting information relating to the patient's need for action relating to ordering medication. This information can include the patient's health status and prescription history. The physician can then analyze the information, determine, for example, whether a prescription should be nullified, voided or otherwise discontinued, indicate that the pharmacy should be contacted to discontinue the prescription, and transmit that information from the provider subsystem 12 to the central database subsystem 11. The central database subsystem 11 can then transmit any information or indications to the product order subsystem 10 of the appropriate pharmacy.

The system allows pharmacists to utilize their extensive knowledge of medication and automatically contact a physician regarding prescriptions. For example, as part of the data feed to the central database subsystem 11, a pharmacist can transmit notes and suggestions relating to patient prescriptions, along with an indication that physicians should be contacted. The central database subsystem 11 can then communicate the pharmacist's notes to appropriate physicians. The physicians can then transmit notes and indications to the central database subsystem 11, that should then be communicated to pharmacists.

The system of present invention provides the advantage of automating the communication between pharmacists and physicians. Since the computer processor 21 of the central database subsystem 11 is programmable, the pharmacist need not indicate that a physician should be contacted, and vice-versa. The processor 21 can be programmed to automatically contact a pharmacist or physician upon determining, based on information stored in the database 24, that a certain condition is met.

## Claims

1. An apparatus for facilitating the process of taking action relating to ordering health-related products, said apparatus comprising:
a central database subsystem further comprising: a memory storage device for storing data containing patient-related health information and information relating to ordering health-related products; and a computer processor having a first transmission device capable of automatically transmitting data to a provider subsystem and a product order subsystem, and capable of receiving data from the provider subsystem;
a provider subsystem comprising: a computer system capable of receiving a data transmission from said first transmission device, allowing the health care provider to examine the data transmitted by the first transmission device, and allowing the health care provider to transmit data to the first transmission device of the central database subsystem; and
a product order subsystem comprising a computer system capable of receiving transmission from said first transmission device.

2. An apparatus as in claim 1, wherein said central database subsystem is capable of analyzing data stored in the memory storage device to determine whether a health care provider should be contacted regarding patient need for action relating to health-related products and, if so, automatically transmitting data to the provider subsystem, and wherein the computer database subsystem is also capable of automatically transmitting data to the product order subsystem upon receiving a transmission from the provider subsystem.

3. An apparatus as in claim 1, wherein the stored patient-related health information includes patient identifier, health-related product prescribed to patient, prescribed schedule for use of prescribed product, and the quantity of prescribed product, contact number for health care provider.

4. An apparatus as in claim 1, wherein the stored information relating to ordering health-related products includes product order entity identifier, and product order entity contact number.

5. An apparatus as in claim 1, wherein the memory storage device contains information relating to the compatibility of health-related products.

6. An apparatus as in claim 1 wherein the memory storage device contains information relating to the price of health related products.

7. An apparatus as in claim 1 wherein the memory storage device contains information relating to health insurance companies.

8. An apparatus as in claim 1 further comprising a data input device for providing data to populate the database, and a coupler to communicate the information from the data input device to the central database subsystem.

9. An apparatus as in claim 1 wherein the memory storage device is a computer disk drive.

10. A method for facilitating the process of taking action relating to ordering health-related products, said method comprising:
storing in a memory storage device of a computer system patient-related health information and information relating to ordering health-related products;
using a computer processor of the computer system to process the patient-related health information and determine whether a health care provider should be contacted regarding the patient's need for action relating to ordering health-related products;
automatically transmitting information to a health care provider over a communications system upon determining that the provider should be contacted regarding the patient's need for action relating to ordering health-related products;
allowing the health care provider to analyze the transmitted information to determine the patient's need for a health related product and transmit information relating to that need back to the computer system over the communications system;
receiving a transmission from the computer system of the health care provider regarding patient need for action relating to a health-related product; and
automatically transmitting information to a product order entity.

11. The method of claim 10, wherein information is automatically transmitted to a product order entity only if the data transmitted by the health care provider indicates patient need for action relating to a health-related product.

12. The method of claim 10, wherein the health care provider can transmit to the computer system an indication that a prescription should be refilled for a patient.

13. The method of claim 10, wherein the health care provider can transmit to the computer system an indication that a prescription should be modified for a patient.

14. The method of claim 10, wherein the health care provider can transmit to the computer system an indication that a prescription should be canceled for a patient.

15. The method of claim 10, wherein the health care provider can transmit to the computer system an indication that a new prescription should be filled for a patient.

16. The method of claim 10, including the step of storing in the memory storage device information relating to the compatibility of health-related products.

17. The method of claim 16, including the step of processing the information in the memory storage device to determine the compatibility of health-related products.

18. The method of claim 10, including the step of storing information relating to the price of health related products.

19. The method of claim 10, including the step of storing information relating to health insurance companies.

20. The method of claim 10, including the step of storing information relating to the price of health related products, including identification of product order entities and the prices they charge for health relate products.

21. The method of claim 20, including the step of determining the product order entity which offers the lowest price for a health related product.

22. The method of claim 21, wherein the product order entity to which information is transmitted is the entity offering the lowest price for a health related product.

23. A method for automatically contacting a physician concerning a patient's need for medication and facilitating the process of taking action relating to ordering medication, said method comprising:
storing in a memory storage device of a computer system patient-related health information and information relating to ordering medication;
using a computer processor of the computer system to process the patient-related health information and determine whether a physician should be contacted regarding a patient's need for action relating to ordering medication;
automatically transmitting information to a physician over a communications system upon determining that the physician should be contacted regarding a patient's need for action relating to ordering medication;
allowing the physician to analyze the transmitted information to determine the patient's need for a refill of previously prescribed medication, modification of the prescription of a previously prescribed medication, discontinuance of previously prescribed medication, or a prescription for a new medication, and transmit information relating to that need back to the computer system over the communications system;
receiving a transmission from the computer system of the physician regarding patient need for action relating to ordering medication; and
automatically transmitting information to a product order entity if the data transmitted by the health care provider indicates patient need for action relating to a health-related product.

24. The method of claim 23, wherein the product order entity is a pharmacy.

25. The method of claim 23, wherein the product order entity is a direct mail house.

26. A method for automatically contacting a physician concerning a patient's need for medication and facilitating the process of taking action relating to ordering medication, said method comprising:
storing in a memory storage device of a computer system patient-related health information and information relating to ordering medication;
using a computer processor of the computer system to process the patient-related health information and determine whether a physician should be contacted regarding a patient's need for action relating to ordering medication;
automatically transmitting information to a physician over a communications system upon determining that the physician should be contacted regarding a patient's need for action relating to ordering medication;
allowing the physician to analyze the transmitted information to determine the patient's need for action relating to ordering a medication, indicate that a pharmacy should be contacted relating to the patient's need for action relating to ordering a medication, and transmit that indication and information relating to that need back to the computer system over the communications system;
receiving a transmission from the computer system of the physician regarding patient need for action relating to ordering medication; and
automatically transmitting information to a pharmacy if the physician indicated that the pharmacy should be contacted.

27. A method for automatically contacting a physician concerning a patient's need for medication and facilitating the process of taking action relating to ordering medication, said method comprising:
receiving from a pharmacy a patient's prescription history information, including patient identifier, the medication prescribed to the patient, and the number of doses dispensed.
storing in a memory storage device of a computer system patient-related health information and information relating to ordering medication;
using a computer processor of the computer system to process the information stored in the memory storage device and determine whether a physician should be contacted regarding a patient's need for action relating to ordering medication;
automatically transmitting information to a physician over a communications system upon determining that the physician should be contacted regarding a patient's need for action relating to ordering medication;
allowing the physician to analyze the transmitted information to determine the patient's need for action relating to ordering a medication, indicate that a pharmacy should be contacted relating to the patient's need for action relating to ordering a medication, and transmit that indication and information relating to that need back to the computer system over the communications system;
receiving a transmission from the computer system of the physician regarding patient need for action relating to ordering medication; and
automatically transmitting information to a pharmacy if the physician indicated that the pharmacy should be contacted.

28. A method for automatically contacting a physician concerning a patient's need for medication and facilitating the process of taking action relating to ordering medication, said method comprising:
storing in a memory storage device of a computer system patient-related health information and information relating to ordering medication, wherein the memory storage device is coupled, via a communications system, to a first communications device which is accessible to a physician and a second communications device which is accessible to a pharmacy;
using a computer processor of the computer system to process the information stored in the memory storage device;
automatically transmitting information to the first communications device of the physician over the communications system;
allowing the physician to analyze the transmitted information and to indicate that a pharmacy should be contacted relating to the patient's need for action relating to ordering a medication, and transmit that indication back to the computer system over the communications system;
receiving a transmission from the first communications device of the physician; and
automatically transmitting information to the second communications device of the pharmacy.
